# EUROPEAN PATENT APPLICATION

(11) **EP 0 631 793 A1**
(43) Date of publication of application: **04.01.1995**
(21) Application number: 94304520.3
(22) Date of filing: 22.06.1994
(51) Int. Cl.: A61M 25/06

(54) **Splittable hemostatic valve and method of use with a splittable introducer sheath**

(30) Priority: 30.06.1993 US 85923
(71) Applicant: Cook Incorporated, Bloomington Indiana 47402 (US)
(72) Inventor: Uldall, Peter R., Willowdale, Ontario M2L 1M2 (CA)
(74) Representative: Johnston, Kenneth Graham

(57) **Abstract**

A splittable hemostatic valve and introducer sheath assembly for introducing a lead or catheter therethrough and into a vessel of a patient. The assembly provides a relatively fluid-tight seal so that the assembly can remain in the vessel of a patient throughout an entire surgical operation with the advantage of devices being exchanged therethrough without bleeding, risk of air embolism, or repeated sheath insertion related trauma associated with such exchanges. The assembly includes a splittable introducer sheath and a splittable hemostatic valve coupled to the introducer sheath arranged and configured to permit introduction of a lead or catheter therethrough. To remove the hemostatic valve or introducer sheath from the implanted lead or catheter, means for permitting removal are provided for splitting apart or peeling away the hemostatic valve and the introducer sheath without the necessity of sliding either the valve or the sheath over the free end of the lead or catheter. The removal means for the hemostatic valve include a pair of diametrically opposed score lines extending along the length of the valve body. The removal means for the introducer sheath comprise a pair of longitudinal tabs along with a folded-back portion extending toward the distal end of the tubular sheath structure. The hemostatic valve assembly further comprises an annular elastic band for maintaining a slit membrane in a closed position, which is positioned proximate the proximal end of the valve body.

## Description

### Technical Field

This invention relates generally to longitudinally splittable or peel-away introducer sheaths and, in particular, to a splittable or peel-away hemostatic valve for use with the introducer sheath and a method of catheterization with the splittable hemostatic valve and introducer sheath.

### Background of the Invention

Introducer sheaths that can be split apart or peeled away for removal from around a medical device in the body of a patient are commonly positioned in an artery of a patient for providing vascular access for medical devices such as guiding, angioplasty, or hemodialysis catheters and pacemaker leads. The Cook Peel-Away™ sheath, for example, is formed of a polytetrafluoroethylene material tube that is longitudinally split to provide a pair of pull tabs at the open, proximal end thereof. A problem with this or other commercially available, splittable introducer sheaths is that excessive bleeding can occur via the open, proximal end of the sheath. Rapid blood loss results in the need for constant clean-up on the part of medical personnel. More importantly, unnecessary or excessive bleeding increases the risk of complications for the patient. For example, continued bleeding increases the likelihood of embolism formation at the access site and clotting inside the sheath. As a result, the sheath is commonly removed and replaced with another sheath. The process of exchanging sheaths results in further patient discomfort and trauma to tissue at the access site. When tissue at the access site is injured during sheath exchange, vascular access must be gained at another anatomical site. Furthermore, exchanging sheaths and/or gaining access at another site prolongs the duration of a surgical procedure.

One suggestion for limiting blood flow from the open, proximal end of a splittable introducer sheath is to manually pinch the sheath closed. A problem with this suggestion is that it is inconvenient to tie up one hand of the physician or other medical personnel throughout the procedure. Another problem with this suggestion is that the sheath is released when introducing another medical device into the proximal end of the sheath. As a result, blood flows from the sheath, and the aforementioned undesirable effects can occur despite efforts to hold the sheath closed.

Another suggestion for limiting blood flow from the open, proximal end of a splittable introducer sheath is to at least partially occlude the sheath lumen with other medical devices inserted therethrough. The practice of introducing through the sheath only medical devices with an outside diameter approximating the inside diameter of the sheath reduces the effective lumen area through which blood loss can occur. As a result, the rate of blood loss through the sheath is decreased. However, blood loss does increase when devices are exchanged in the sheath. Furthermore, the size of the inserted device must be matched to the inside diameter of the sheath. In keeping with this approach, a number of splittable introducer sheaths are commercially available in a set with a cooperating dilator having, for example, an outside diameter in close tolerance to the inside diameter of the sheath.

Still another suggestion for limiting blood flow from the open, proximal end of a splittable introducer sheath is to attach or couple a seal or valve to the proximal end thereof. However, known seal or valve structures for a splittable introducer sheath typically require removal over the free end of the implanted device disposed therethrough. These seal or valve structures may be suitable for their intended purposes, but are unacceptable when a termination at the end of the implanted lead or catheter is larger in diameter than that of the valve and seal, and the lead or catheter must remain implanted.

### Summary of the Invention

The foregoing problems are solved and a technical advance is achieved in an illustrative splittable hemostatic valve and introducer sheath assembly for use with a lead or catheter disposed therethrough without removing the hemostatic valve or introducer sheath over the free end of the lead or catheter. The valve and sheath assembly comprises a splittable introducer sheath and a splittable hemostatic valve coupled to the introducer sheath, which are arranged and configured to permit the introduction of a lead or catheter therethrough. As a result, the assembly effectively minimizes, if not practically eliminates, the flow of blood therefrom and the inherent risks associated therewith. Furthermore, the valve and sheath assembly also comprises means for permitting removal of the hemostatic valve and introducer sheath from the lead or catheter disposed therethrough without requiring the introducer sheath and hemostatic valve to be removed from the free end of the lead or the catheter.

As a result, the assembly may safely remain in the vessel lumen throughout a percutaneous medical procedure without substantial bleeding, risk of air embolism, clotting, or need of repeated sheath insertion for lead exchange.

In one aspect, the means for permitting removal of the hemostatic valve and introducer sheath is a means for splitting the introducer sheath and hemostatic valve away from the lead or catheter disposed therethrough.

In another aspect, the means for permitting removal of the hemostatic valve and introducer sheath is a means for peeling away the introducer sheath and hemostatic valve away from the lead or catheter disposed therethrough.

In still another aspect, the means for permitting removal of the hemostatic valve and introducer sheath is a score line and, in particular, a pair of diametrically opposed score lines disposed along the longitudinal length of and in the hemostatic valve along which the hemostatic valve may be separated.

In the preferred embodiment, the hemostatic valve of the assembly comprises a valve body including first and second body sections that are splittable longitudinally apart from each other to effect removal of the valve from the introducer sheath and the lead or catheter disposed therethrough. The body sections include a pair of diametrically opposed flanges, such as pull tabs, positioned proximate the proximal end thereof for facilitating advantageous separation of the body sections. The hemostatic valve includes a slit extending from the proximal end of the valve and communicating with the pair of score lines for assuring that the hemostatic valve is split along its entire length.

The hemostatic valve further includes a chamber extending longitudinally in the valve body for reception of the introducer sheath. The chamber has an open distal end and a proximal end within a specified distance of the proximal end of the valve body. A valve membrane is positioned between the proximal ends of the chamber and the valve body. A passage extends through the valve membrane and communicates with the chamber of the hemostatic valve. This passage advantageously permits introduction of a guidewire or a lead therethrough and into the chamber while maintaining a relatively fluid-tight seal between the valve body and the guidewire or lead. The slit extending in the valve body also passes through the centerline of the guidewire passage.

To maintain the valve membrane and splittable passage in a closed position, the hemostatic valve also includes an annular elastic band that is positioned in an annular channel extending around the valve body. The elastic band maintains a tight seal between the valve body and the end of the introducer sheath inserted in the chamber. A pull tab extends from the annular band to facilitate its severance during removal of the valve and introducer sheath assembly.

The means for splitting the introducer sheath of the assembly comprises a tubular structure having at one end first and second longitudinal tabs. The tabs and a portion of the tubular structure adjacent the tabs are advantageously folded back over the tubular structure and extend toward the opposite distal end of the tubular structure to facilitate separate removal of the introducer sheath. When the tabs are pulled apart, the tubular structure tears longitudinally apart along its entire length, thereby facilitating removal of the introducer sheath from the lead or the catheter disposed therethrough without requiring removal of the introducer sheath from an end of the lead or catheter. Advantageously, the valve and sheath of the assembly in one aspect are separate parts which are then coupled to each other. In addition, the means for permitting removal of the assembly from a lead or catheter disposed therethrough allow separate removal of the valve and sheath from the lead or catheter as desired by the physician.

The invention may also be characterized as a splittable hemostatic valve assembly for use with an introducer sheath. The assembly comprises a valve body having score lines extending longitudinally therealong for splitting the valve body longitudinally apart. Flanges extend from the valve body for pulling the valve body longitudinally apart along the score lines. A chamber extends longitudinally in the valve body and has an open distal end for reception of the introducer sheath. A slit extends from the proximal end of the valve body and into a proximal end of the chamber and score lines. To further facilitate slitting the valve body, the valve assembly includes first and second body sections and splittable walls for interconnecting the body sections together proximate the score lines.

To facilitate maintaining a fluid-tight seal between the valve assembly and a guidewire, lead, or catheter disposed therethrough, the valve assembly comprises a membrane positioned between the proximal ends of the chamber and the valve body. A passage extends through the membrane for advantageously introducing a guidewire or a lead through the assembly. The valve body also includes an annular channel positioned therearound and proximate the proximal end of the chamber. The valve assembly also includes a band with a pull tab that is positioned around the valve body and in the annular channel to further maintain the relatively fluid-tight seal. To further assure splitting the valve body longitudinally apart, the slit and the score lines extend into the annular channel.

The invention is also characterized as a method of percutaneous catheterization comprising the steps of providing an introducer sheath, a hemostatic valve, and means for permitting removal of the hemostatic valve and the introducer sheath from a lead or a catheter disposed therethrough without requiring the introducer sheath and the hemostatic valve to be removed from an end of the lead or the catheter. The hemostatic valve is coupled to the introducer sheath, which is configured to permit introduction of the lead or catheter therethrough. The introducer sheath and hemostatic valve coupled thereto is then disposed in a body lumen, after which the lead or catheter is positioned through the valve and sheath into the body lumen. The hemostatic valve is removed from the introducer sheath and the lead or catheter disposed therethrough without removing the hemostatic valve over the end of the lead or catheter disposed therethrough. After removal of the hemostatic valve, the introducer sheath is then removed from the lead or catheter disposed therethrough without removing the introducer sheath over an end of the lead or catheter.

The step of removing the hemostatic valve includes splitting apart or peeling away the hemostatic valve longitudinally from the introducer sheath and the lead or catheter disposed therethrough.

The step of removing the introducer sheath includes splitting the introducer sheath longitudinally apart and away from the lead or catheter disposed therethrough after the hemostatic valve has been removed.

The invention is still further characterized as an improvement in an introducer sheath and valve assembly for implantation of pacemaker lead comprising an element for splitting the introducer sheath. The sheath has a longitudinal axis. The element for splitting allows manual separation of the sheath along the longitudinal axis. An other element for separating the hemostatic valve permits removal of the valve from the lead without necessitating removal of the valve over an end of the lead. As a result, the sheath can remain in place throughout the operation with the advantage of free lead exchange and easier lead

The word splitting includes within its meaning the formation of either a single longitudinal slit along a tube, seal or sheath, or a plurality of slits therealong. In the case of a single slit, the sheath or seal can be unfolded so that it can be laterally removed. In the case of two diametrically opposed slits being formed, the two resulting parts can be separated and laterally removed. In the case of three equally spaced slits, the separated parts can also be removed laterally.

The slit can be formed by scoring the surface of a material or by forming a weakened region in the material such that when a force is applied to the material it splits in a longitudinal direction. The slit can be linear or curved as desired, providing it extends in a longitudinal direction. manipulation without bleeding, air embolism, clotting, and repeated sheath related trauma for possible lead exchange.

### Brief Description of the Drawing

FIG. 1 depicts a partially sectioned, pictorial view of a splittable hemostatic valve and introducer sheath assembly of the present invention;
FIG. 2 depicts a pictorial view of the splittable introducer sheath of the assembly of FIG. 1;
FIG. 3 depicts a partially sectioned side view of the assembly of FIG. 1;
FIG. 4 depicts a pictorial view of the annular elastic band of the assembly of FIG. 1;
FIG. 5 depicts a distal end view of the valve body of the assembly of FIG. 1;
FIG. 6 depicts an enlarged, partially sectioned side view of a valve body section of FIG. 3;
FIG. 7 depicts an elevated, partially sectioned, pictorial view of the assembly of FIG. 1;
FIG. 8 depicts a partially sectioned, pictorial view of the assembly of FIG. 1 with just a guidewire extending therethrough; and
FIGs. 9 and 10 depict a method of percutaneous catheterization including the assembly of FIG. 1.

### Detailed Description

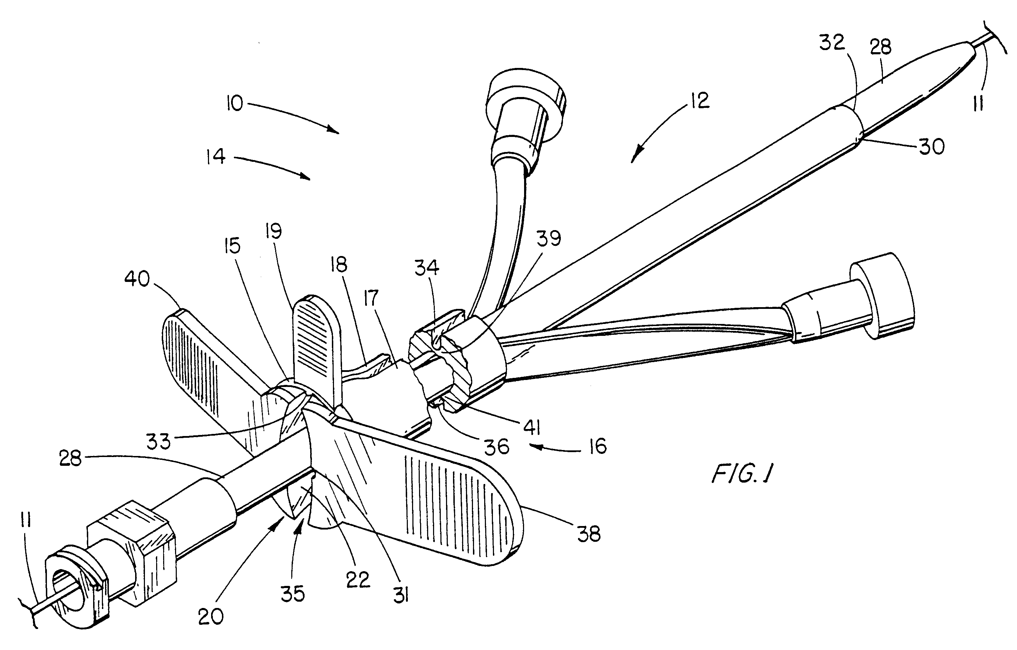

FIG. 1 depicts a partially sectioned, pictorial view of an illustrative embodiment of splittable hemostatic valve and introducer sheath assembly 10 with guidewire 11 and dilator 28 coaxially positioned therethrough for introducing leads or catheters through the combined valve and sheath assembly into a vessel of a patient. The valve and sheath assembly comprises a splittable introducer sheath 12 connected or coupled to or extending from a splittable hemostatic valve assembly 14. When the hemostatic valve and introducer sheath are removed from the implanted lead or catheter which must remain implanted, means are employed to split, peel away, or separate the hemostatic valve and introducer sheath longitudinally apart so that the valve and sheath are removed from the implanted lead catheter without sliding either the valve or sheath over the free proximal end of the lead or catheter. As a result, any termination that is provided on the free proximal end of the lead or catheter will not interfere with the removal of the hemostatic valve and introducer sheath assembly. Since the hemostatic valve and introducer sheath are separate parts which are then coupled together in the preferred embodiment, the means for permitting removal of the assembly from a lead or catheter disposed therethrough allow for separate removal of the valve and the sheath from the lead or catheter as desired by the attending physician. Furthermore, the valve and sheath assembly may remain in a vein throughout a surgical procedure or operation with the advantage of free lead exchange and easier lead manipulation without substantial bleeding, risk of air embolism, clotting, or repeated sheath insertion related trauma due to lead or catheter exchanges.

Splittable hemostatic valve assembly 14 comprises valve body 16 with diametrically opposed score lines 34 and 36 such as channels extending longitudinally therealong and into the valve body. The valve body includes body sections 17 and 18 that are interconnected by splittable walls 39 and 41 at the bottom of score line channels 34 and 36, respectively. Body sections 17 and 18 are splittable longitudinally apart from each other through score line channels 34 and 36. Slit 35 extends through valve membrane 22 from proximal end 20 of the valve assembly and into score line channels 34 and 36 to further ease longitudinal separation of the body sections. To maintain valve membrane 22 in a closed position about slit 35, valve assembly 14 further includes an elastic band 15 positioned around valve body 16 and in annular channel 33 proximate proximal end 20 of the valve body. Pull tab 19 extends laterally from elastic band 15 for stretching and cutting the band just prior to longitudinally splitting or peeling apart the body sections of the valve body.

Splittable valve body 16 also includes diametrically opposed flange portions 38 and 40 positioned proximate proximal end 20 of the valve body and extending laterally from body sections 17 and 18, respectively. After annular elastic band 15 is removed, flange portions 38 and 40 are diametrically pulled apart to open slit 35 and longitudinally split apart or peel away body sections 17 and 18 of valve body 16 through score line channels 34 and 36.

In the depiction of FIG. 1, a well-known and commercially available dilator 28 is shown disposed through valve assembly 14 and introducer sheath 12. The sheath has a tapered distal tip 30 extending from distal end 32 thereof.

As is known with the well-known Seldinger percutaneous introduction technique, a vessel is punctured with a needle into which a guidewire is inserted therethrough. The needle is removed, and then dilator 28 with splittable valve and sheath assembly 10 positioned therearound is advanced over the guidewire and into the vessel. Coaxially positioned guidewire 11 and dilator 28 spread apart and open slit 35 and valve membrane 22 when positioned through valve and sheath assembly 10. The guidewire and dilator when positioned through introducer sheath 12 form a seal to minimize the loss of blood, if any, through the introducer sheath. After the valve and sheath assembly are introduced into a vessel, dilator 28 is removed from the vessel and the valve and sheath assembly, thus leaving the valve and sheath assembly in place along with the guidewire inserted therethrough. Passage 31 extending through valve membrane 22 forms a near fluid-tight seal around guidewire 11 after dilator 28 has been removed.

Annular elastic band 15 of the valve assembly maintains slit 35 and membrane 22 in a closed position around the guidewire to minimize bleeding. At this stage of the percutaneous surgical procedure, one or more catheters such as a hemodialysis catheter can be introduced, removed, and reintroduced and manipulated without any significant bleeding, clotting, risk of air embolism or repeated sheath insertion related trauma since valve and sheath assembly 10 remains in position during the procedure. When, for example, inserting a collapsible lumen hemodialysis catheter such as disclosed in U.S. Patent No. 5,106,368 of the present inventor, an obturator is inserted into one of the lumens of the catheter and inserted over the guidewire and through the introducer sheath into a jugular or femoral vein. The collapsible lumen hemodialysis catheter opens slit 35 of the valve assembly, but the elastic band maintains a tight seal of the valve body against the introducer sheath to minimize any bleeding through the valve and sheath assembly. Alternatively, when, for example, inserting a pacemaker lead, the diameter of splittable passage 31 through valve membrane 22 can be changed, if not eliminated, to form a seal with the pacemaker lead and the proximal end of the introducer sheath and prevent loss of blood therethrough. After the guidewire and dilator are removed, the pacemaker lead is inserted through slit 35 and passage 31, if any, extending through valve membrane 22.

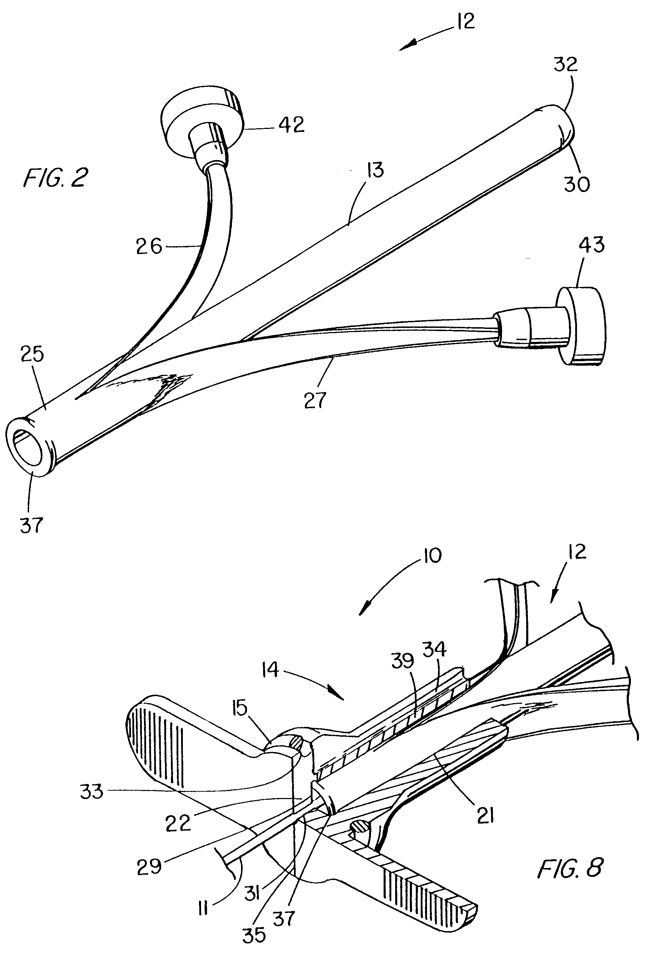

FIG. 2 depicts a pictorial view of splittable introducer sheath 12 of assembly 10 of FIG. 1. Splittable introducer sheath 12 is commercially available from Cook Incorporated, Bloomington, Indiana, and is disclosed in U.S. Patent No. 4,306,562 and reissued U.S. Patent No. RE 31,855 of T. A. Osborne, which are incorporated by reference herein. Splittable sheath 12 comprises a tubular structure 13 having tapered tip 30 extending proximally from the distal end 32 thereof. The tubular structure is formed of a flexible material such as polytetrafluoroethylene having the physical property of molecular orientation, whereby a tear in the flexible material runs readily only in a longitudinal direction along the length of the tubular structure. The proximal end of the tubular structure has a pair of open-ended slits, thus forming longitudinal tabs 26 and 27 with respective knobs 42 and 43 affixed thereto for ease in longitudinally splitting apart or peeling away the sheath from a lead or catheter disposed therethrough. The longitudinal tabs along with adjacent portion 25 of the tubular structure are folded back over the tubular structure toward distal end 32 of the tubular structure, thus forming open end 37 for insertion into the open distal end of splittable hemostatic valve assembly 14. By way of example, sheath 12 has an inside diameter of 13 French (.170'') and an outside diameter of approximately .194''. Folded-back portion 25 has an outside diameter of approximately .214''. Folded-back end 37 has an outside diameter of approximately .224''.

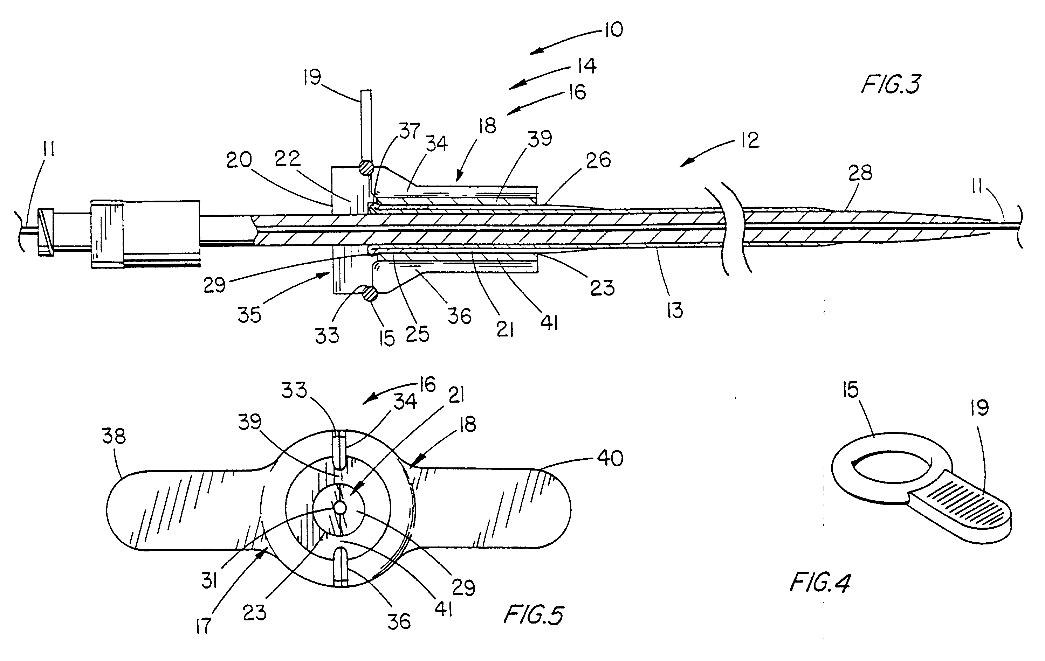

Depicted in FIG. 3 is a partially sectioned side view of splittable valve and sheath assembly 10 of FIG. 1 with guidewire 11 and dilator 28 coaxially positioned through splittable hemostatic valve assembly 14 and splittable introducer sheath 12. As depicted, folded-back end 37 of the introducer sheath along with folded-back portion 25 is inserted through open distal end 23 of valve body 16 and into chamber 21 extending longitudinally therein. The folded-back end of the sheath is pushed through chamber 21 and abuts against proximal end 29 of the chamber. The approximately .207'' diameter of the chamber is sized to form a seal with the folded-back end and portion of the sheath when positioned in chamber 21. This minimizes any bleeding or the introduction of air between the slip or friction fit coupling of the splittable valve body and the introducer sheath.

Hemostatic valve 14 also includes an annular channel 33 positioned circumferentially around valve body 16 proximate proximal end 20 of the valve body for positioning elastic band 15 therein. The centerline of the channel is positioned to coincide with proximal end 29 of chamber 21. With elastic band 15 positioned in annular channel 33, valve membrane 22 with slit 35 therethrough is maintained in a closed position even with folded-back end 37 of the introducer sheath abutted against the membrane and proximal end 29 of the chamber. Slit 35 extends from proximal end 20 of the valve assembly through valve membrane 22 and a short distance into splittable walls 39 and 41 of chamber 21. Slit 35 also communicates with score line channels 34 and 36 and annular channel 33. The extension of slit 35 into score line channels 34 and 36 and proximal end 29 of the chamber assures that body sections 17 and 18 can be longitudinally split apart through score line channels 34 and 36. Slit 35 communicates with the proximal ends of splittable walls 39 and 41 to assure that body sections 17 and 18 split longitudinally apart along their entire length and peel away from the proximal end of the introducer sheath.

By way of example, valve body 16 is approximately 1.000'' long with a diameter of approximately .400'' along the distal portion which increases to a diameter of approximately .600'' in the vicinity of proximal end 20. Cylindrically-shaped chamber 21 is centered along the longitudinal axis of the valve body and has a diameter of approximately .207'' extending from open distal end 23 to proximal chamber end 29. Proximal chamber end 29 is positioned a specified distance approximately .190'' from proximal end 20 of the valve body, thus making valve membrane 22 of the same thickness. Splittable walls 39 and 41 are approximately .026'' thick. Each of diametrically opposed score line channels 34 and 36 extends longitudinally along the distal portion of the valve body and is approximately .045'' wide with a U- or V-shaped bottom therealong.

Depicted in FIG. 5 is a distal end view of valve body 16 with chamber 21 extending longitudinally therein from open distal end 23. Splittable passage 31 is coincident with the centerline of the valve body and chamber 21. Slit 35 extends through the valve membrane and is also coincident with the centerline of the valve body. Body sections 17 and 18 are interconnected by splittable walls 39 and 41. Score line channels 34 and 36 each extend transversely down approximately .045'' from the outer surface of the valve body and then angle toward splittable walls 39 and 41, respectively. Annular channel 33 extends approximately .040'' from the outer surface and into the valve body. Flange portions 38 and 40 are approximately .330'' wide and extend approximately .900'' from the centerline of the valve body. The flanges are approximately .095'' in overall thickness.

Returning to FIG. 3, slit 35 extends to a depth of approximately .225'' from proximal end 20 of valve body 16. As a result, slit 35 extends approximately .035'' into the proximal end of chamber 21 and the proximal ends of splittable walls 39 and 41. Slit 35 transversely extends almost entirely through annular channel 33, which is an approximately .040'' radiused channel extending circumferentially around the valve body. The centerline of the annular channel is also approximately .190'' from proximal end 20 of the valve body and coincides with proximal end 29 of chamber 21. Score line channels 34 and 36 extend partially into annular channel 33 approximately .200'' from proximal end 20 of the valve body.

Depicted in FIG. 4 is a pictorial view of annular elastic band 15 with pull tab 19 extending laterally therefrom. Elastic band 15 and pull tab 19 are molded in a well-known manner from a medical grade silicone having a durometer of 50 on the Shore A Scale. The elastic band has a cross-sectional diameter of approximately .075'' with an inside diameter of approximately .297''. Pull tab 19 is approximately .220'' wide and approximately .425'' long from the inside diameter of the elastic band. The overall thickness of the tab is approximately .095''.

FIG. 6 depicts an enlarged, partially sectioned side view of hemostatic valve body section 18 of FIG. 3 without guidewire 11, introducer sheath 12, and dilator 20 coaxially positioned therethrough and in chamber 21. Chamber 21 extends longitudinally in the body section and opens at distal end 23. Slit 35, as previously described, extends from proximal body end 20 through valve membrane 22 and proximal chamber end 29 and into chamber 21, annular channel 33, score line channels 34 and 36, and splittable walls 39 and 41. Sectioned splittable walls 39 and 41 indicate where the valve body is longitudinally split apart. Elastic band 15 is positioned in annular channel 33. Splittable passage 31 extends from proximal assembly end 20 to proximal chamber end 29 through valve membrane 22. Splittable passage 31 is approximately .042'' in diameter for the positioning of an approximately .038'' guidewire therethrough. Slit 35 is coincident with the centerline of splittable passage 31. Valve 16 is molded in a well-known manner using, for example, medical grade silicone having a durometer of 25 on the Shore A Scale.

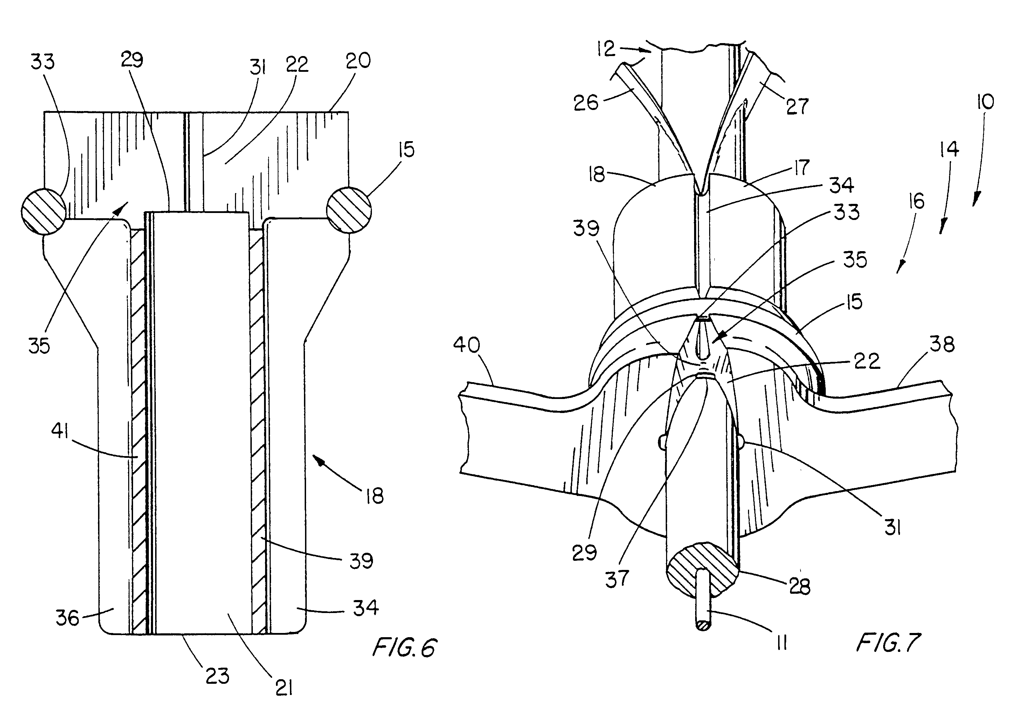

FIG. 7 depicts an elevated, partially-sectioned, pictorial, view along the lineal axis of valve and sheath assembly 10 from the proximal end thereof, with guidewire 11 and dilator 28 coaxially positioned through introducer sheath 12 and valve assembly 14. Introducer sheath 12 is positioned in the chamber of valve body 16 with folded-back end 37 of the sheath abutted against proximal chamber end 29. With guidewire 11 and dilator 28 coaxially positioned through valve and sheath assembly 10, slit 35 is partially open, causing valve membrane 22 to be spread apart. However, elastic band 15 in annular channel 33 forces score line channels 34 and 36 and slit 35 individually inward to prevent valve body 16 from being split apart and to maintain the seal of chamber 21 against introducer sheath 12. When dilator 28 is removed from the valve and sheath assembly, elastic band 15 maintains slit 35 and valve membrane 22 in a closed position. Splittable passageway 31 then surrounds and forms a seal around guidewire 11.

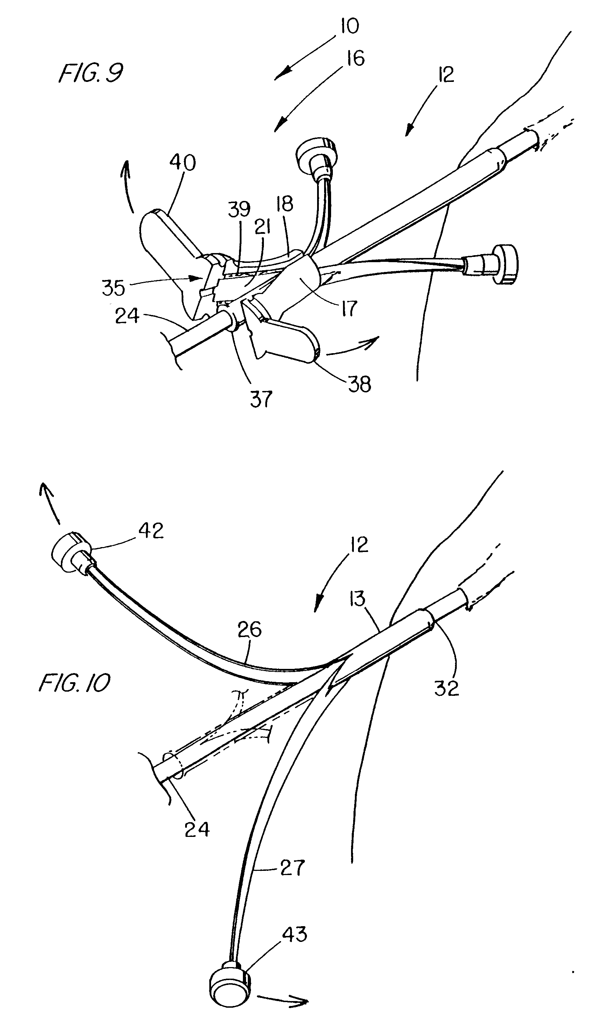

FIG. 8 depicts a partially sectioned, pictorial view of valve and sheath assembly 10 with just guidewire 11 coaxially positioned through valve assembly 14 and splittable sheath 12. Elastic band 15 in annular channel 33 maintains slit 35 and valve membrane 22 in a closed position around guidewire 11. Since slit 35 extends into chamber 21, the centerline of elastic band 15 is positioned coincident with proximal chamber end 29 to maintain a fluid-tight seal against folded-back sheath end 37. Slit 35 extends into chamber 21 as well as score line channels 34 and 36 and splittable walls 39 and 41 to assure that valve assembly 14 is longitudinally split apart and peeled away from introducer sheath 12 and any catheter or lead coaxially positioned therethrough. Experiments have indicated that the positioning of elastic band 15 coincident with proximal chamber end 29 is critical in maintaining a fluid-tight seal with introducer sheath 12.

FIGs. 9 and 10 depict the method of percutaneous catheterization including the separate peel-away removal of valve assembly 14 from introducer sheath 12 with lead or catheter 24 coaxially positioned therethrough and the subsequent peel-away removal of splittable introducer sheath 12. These separate peel-away operations are performed without removing the hemostatic valve assembly or introducer sheath over an end of the lead or catheter disposed therethrough. According to the invention, both valve assembly 14 and introducer sheath 12 are splittable longitudinally or have a peel-away construction. Introducer sheath 12 and valve assembly 14 are shown as separate pieces that are coupled together by inserting folded-back end 37 of introducer sheath 12 into chamber 21 of valve body 16. Although shown as separately fabricated and coupled together, introducer sheath 12 and valve assembly 14 can be integrally fabricated. As shown in FIG. 9, lead or catheter 24 has been percutaneously inserted into the vessel of a patient through splittable valve and introducer sheath assembly 10 using the well-known Seldinger technique. To remove valve assembly 14 from introducer sheath 12, elastic band 15 is first cut and removed from the valve assembly. The physician then grasps flange portions 38 and 40 of valve body 16 and outwardly pulls them apart. As shown, splittable walls 39 and 41 are split longitudinally along their entire length, and body sections 17 and 18 are peeled away from introducer sheath 12 and catheter 24.

After the valve assembly has been removed from the introducer sheath, the physician then grasps longitudinal tabs 26 and 27 of the introducer sheath with knobs 42 and 43 and outwardly pulls on them to continue separation of the closed-ended slits in the sheath along the folded-back portion of the sheath. After the folded-back portion is split, continual outward pulling separates the remaining length of tubular structure 13 toward distal end 32. As a result, the introducer sheath is peeled away from catheter 24 into two pieces without having to extend the sheath over the free end of the catheter.

It is to be understood that the above-described splittable hemostatic valve and introducer sheath assembly is merely an illustrative embodiment of the principles of this invention and that other embodiments may be devised by those skilled in the art without departing from the spirit and scope of this invention. Any combination of a splittable hemostatic valve in combination with an introducer sheath, whether integrally formed or separate parts which are then coupled together, is contemplated by the present invention. Furthermore, the splittable hemostatic valve assembly in combination with an introducer that is either unsplittable or splittable longitudinally is contemplated.

## Claims

1. An arrangement for forming a seal at the proximal end of an introducer sheath (12), said arrangement comprising a tubular assembly (16) for supporting or to be coupled to the introducer sheath and for permitting a lead or instrument (11,28,13) to be introduced through the sheath into a patient, characterized in that the tubular assembly has a structure which enables the assembly to be longitudinally split and to be laterally withdrawn with respect to the said instrument.

2. An arrangement according to claim 1, wherein the said structure enables the assembly to be split along first and second potential fracture lines (34,36), whereby the assembly can be split into two separate parts (17,18) capable of being removed laterally with respect to the longitudinal axis of the said instrument.

3. An arrangement according to claim 1 or 2, wherein the introducer sheath is supported by the tubular assembly, and wherein the introducer sheath also has a structure which enables it to be longitudinally split and laterally withdrawn.

4. An arrangement according to claim 3, wherein the said tubular assembly comprises a chamber (21) longitudinally extending therein, and wherein the introducer sheath is to be contained and sealed within the chamber.

5. An arrangement according to claim 4, wherein a clamping arrangement (15) extends around or adjacent to the proximal end (20) of the tubular assembly to form the seal.

6. An arrangement according to claim 5, wherein a slit (35) extends from the proximal end of the tubular assembly, through a membrane (22) into the proximal end of the said chamber, and wherein the distal end of the slit coincides with the said structure of the assembly.

7. An arrangement according to any one preceding claim, wherein the said structure comprises one or more score lines (34,36) in the surface of the assembly and/or the introducer sheath.

8. An arrangement according to claim 5, wherein the clamping arrangement comprises a peripheral groove (33) around the proximal end of the assembly, and a band (15) in the groove for exerting an inwardly directed force.

9. An arrangement according to any preceding claim, wherein the assembly comprises first and second body sections (17,18) that are splittable apart, and wherein each section has a flange (38,40) extending therefrom to facilitate the splitting action.

10. An arrangement according to claim 7, wherein the introducer sheath also comprises a pair of tabs (42,43) which enable the sheath to be split along the score lines after the assembly has been removed.

11. An assembly for use with a lead or catheter, comprising an introducer sheath; a seal or valve coupled to said introducer sheath, said seal or valve and introducer sheath being arranged and configured to permit introduction of at least one lead or catheter therethrough; and
means for permitting removal of said seal or valve and introducer sheath from said lead or catheter disposed therethrough without requiring said introducer sheath and seal or valve to be removed from an end of said lead or catheter,
whereby said assembly may remain in a vein throughout an operation with the advantage of free lead exchange and easier lead manipulation without substantial bleeding, risk of air embolism, clotting or repeated sheath insertion related trauma from lead exchange.
